(19) Europäisches Patentamt / European Patent Office / Office européen des brevets

(11) **EP 3 721 862 B1**

(12) **EUROPEAN PATENT SPECIFICATION**

(45) Date of publication and mention of the grant of the patent:
**24.07.2024 Bulletin 2024/30**

(21) Application number: **20172577.7**

(22) Date of filing: **13.01.2020**

(51) International Patent Classification (IPC):
***A61K 8/36*** *(2006.01)* ***A61K 8/43*** *(2006.01)*
***A61K 8/44*** *(2006.01)* ***A61Q 11/00*** *(2006.01)*
***A61K 9/08*** *(2006.01)* ***A61K 47/36*** *(2006.01)*
***A61K 9/00*** *(2006.01)* ***A61K 31/155*** *(2006.01)*

(52) Cooperative Patent Classification (CPC):
(C-Sets available)
**A61Q 11/00; A61K 8/361; A61K 8/43; A61K 8/44; A61K 31/155; A61K 31/198** (Cont.)

(54) **ORAL CARE PREPARATIONS COMPRISING CHLORHEXIDINE AND ARGININE OR THEIR SALTS**

MUNDPFLEGEPRÄPARATE ENTHALTEND CHLORHEXIDIN UND ARGININE ODER IHRE SALZE

PRÉPARATIONS DE SOINS BUCCAUX COMPRENANT DE LA CHLORHEXIDINE ET DE L' ARGININE OU LEURS SELS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **11.01.2019 EP 19151526**

(43) Date of publication of application:
**14.10.2020 Bulletin 2020/42**

(62) Document number(s) of the earlier application(s) in accordance with Art. 76 EPC:
**20700704.8 / 3 697 380**

(73) Proprietor: **Meda AB**
**170 73 Solna (SE)**

(72) Inventors:
- **FILIPPI, Elisabetta**
  **17073 Solna (SE)**
- **ZANARDI, Andrea**
  **17073 Solna (SE)**
- **GELFI, Elena**
  **17073 Solna (SE)**

(74) Representative: **Elkington and Fife LLP**
**Prospect House**
**8 Pembroke Road**
**Sevenoaks, Kent TN13 1XR (GB)**

(56) References cited:
**WO-A1-2017/096000** **US-A1- 2015 313 813**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**A61K 31/155, A61K 2300/00;**
**A61K 31/198, A61K 2300/00**

## Description

This invention relates to a liquid oral care composition that is a mouthwash, mouthrinse or a spray comprising chlorhexidine or a salt thereof which is effective in preventing or reducing symptoms associated with halitosis.

## BACKGROUND OF THE INVENTION

Oral hygiene is an important consideration in the daily life of children and adults around the globe. The maintenance of good oral hygiene is vital to keep the mouth, teeth and gums free from disease, and to prevent the growth of bacteria which can lead to the development of halitosis (bad breath).

Bacteria located in periodontal pockets and at the back of the tongue break down sulfur-containing amino acids, in particular cysteine and methionine, to produce the volatile sulfur compounds, including hydrogen sulfide and methyl mercaptan.

These volatile sulfur compounds have an extremely unpleasant odor, even at low concentrations, and also penetrate the epithelium and cause damage to cells of the underlying tissue. It has also been suggested that volatile sulfur compounds produced by bacteria located within periodontal pockets may be an important factor in the development of periodontal disease.

Therefore, antibacterial agents are often used in preventive dentistry to reduce the levels of these bacteria and thereby prevent the formation of volatile sulfur compounds. One known antibacterial agent used in preventative dentistry is chlorhexidine, which is frequently formulated in a mouthwash.

WO 00/051559 discloses a mouthwash comprising chlorhexidine and zinc acetate, wherein the combination of zinc and chlorhexidine synergistically reduces the production of volatile sulfur compounds.

EP0181161 discloses a fluoride-free oral hygiene product containing a solution of a cationic bis-biguamide antiseptic such as chlorhexidine and zinc ions, wherein the zinc ions are provided by soluble zinc salts of an aldonic acid such as gluconic acid. It also discloses that the stability of the oral hygiene product can be improved if the chlorhexidine and the zinc species share a common anion.

Unfortunately, the applicant has surprisingly discovered that chlorhexidine is unstable in the mouthwash, and degrades overtime. Such degradation is clearly undesirable because the efficacy of the product will be reduced and undesired degradation products form in a product ingested by humans.

Accordingly, it is an object of the present invention to provide a liquid oral care composition comprising chlorhexidine wherein the chlorhexidine has improved chemical stability.

## SUMMARY OF THE INVENTION

In a first aspect, the invention provides a liquid oral care composition that is a mouthwash, mouthrinse or a spray comprising:

- 0.005 to 1 % w/w chlorhexidine or a salt thereof;
- 0.1 to 1% w/w arginine or a salt thereof; and
- a zinc salt.

In a second aspect, the invention provides the liquid oral care composition that is a mouthwash, mouthrinse or a spray as defined by the first aspect of the invention for use in therapy.

Surprisingly, the applicant has found that arginine improves the chemical stability of chlorhexidine in a liquid oral care composition that is a mouthwash, mouthrinse or a spray.

Additional aspects of the invention are more fully described in the following detailed description of the various embodiments.

## DEFINITIONS

The proportions of each component of the liquid composition that is a mouthwash, mouthrinse or a spray used herein are defined as "% w/w", i.e. weight by weight, which is calculated by the following formula:

$$\% \text{ w/w} = [(\text{weight of solute})/(\text{total weight of solution})] * 100.$$

If the relevant solute is added in the form of a salt, the weight of the solute is the weight of the salt before it is added to the liquid composition i.e. the anion(s) and the cation(s).

For the avoidance of doubt, each component in the composition may comprise one member of that class of components or more than one member of that class of component. For example, the chlorhexidine or a salt thereof can comprise a mixture of chlorhexidine and one or more salts of chlorhexidine, the arginine or a salt thereof can comprise a mixture of arginine and one or more salts of arginine, and the zinc salt can comprise a mixture of more than one zinc salts.

## DETAILED DESCRIPTION OF THE INVENTION

### Compositions

The invention provides a liquid oral care composition that is a mouthwash, mouthrinse or a spray comprising:

- 0.005 to 1% w/w chlorhexidine or a salt thereof;
- 0.1 to 1% w/w arginine or a salt thereof; and
- a zinc salt.

Chlorhexidine

The liquid oral care composition that is a mouthwash, mouthrinse or a spray contains chlorhexidine or a salt thereof. Chlorhexidine is a bis-biguanide with the chemical name N,N"-bis(4-chlorophenyl)-3,12-diimino-2,4,11,13-etraazatetradecanediimidamide):

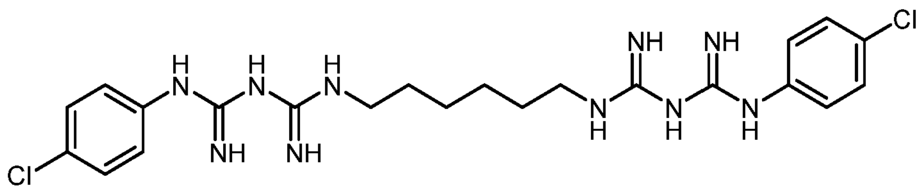

Chlorhexidine is an antibacterial agent which is commonly used in oral care compositions.

In one embodiment of the invention, the composition includes a salt of chlorhexidine, for example a salt with acetic acid (e.g. chlorhexidine diacetate), a salt with hydrogen chloride (e.g. chlorhexidine dihydrochloride) or a salt with gluconic acid (e.g. chlorhexidine digluconate), or mixtures thereof.

The composition comprises 0.005 to 1% w/w chlorhexidine or a salt thereof.

In one embodiment, the composition comprises 0.005 to 0.5% w/w, 0.01 to 0.3% w/w, 0.01 to 0.08% w/w, 0.01 to 0.05% w/w, or 0.02 to 0.04% w/w chlorhexidine or a salt thereof. In particular, the composition includes 0.02 to 0.04% w/w e.g 0.025% w/w chlorhexidine or a salt thereof.

In particular, the composition includes chlorhexidine diacetate. In one embodiment, the composition comprises 0.005 to 1% w/w, 0.005 to 0.5% w/w, 0.005 to 0.1% w/w, 0.01 to 0.3% w/w, 0.01 to 0.08% w/w, 0.01 to 0.05% w/w, or 0.02 to 0.04% w/w chlorhexidine diacetate. In particular, the composition includes 0.02 to 0.04% w/w chlorhexidine diacetate e.g 0.03% w/w chlorhexidine diacetate.

Chlorhexidine is known to have a prolonged and broad spectrum antimicrobial effect, and also to have plaque inhibitory potential (Vrani "Formulation ingredients for toothpastes and mouthwashes" Bosnian Journal of Basic Medical Sciences 2004, 4, 51-58). The antimicrobial effect reduces offensive oral odor caused by VSCs (N. Lourith "Oral malodour and active ingredients for treatment" International Journal of Cosmetic Science 2010, 32, 321-329).

Chlorhexidine is an amphipathic molecule with hydrophilic and hydrophobic groups. At physiological pH, chlorhexidine is cationic and exhibits its antimicrobial activity as a "membrane-active agent" that binds to negatively charged bacterial cell walls. Chlorhexidine establishes a bridge between pairs of adjacent phospholipids within the cell wall and displaces the associated divalent cations, thereby increasing the permeability of the cell membrane, facilitating the release of intracytoplasmic material and ultimately resulting in cell death. The cationic molecule will bind principally to anionic compounds within the cell wall, such as free sulphates, lipopolysaccharide phosphate groups and protein carboxyl groups (Rölla and Melsen "On the mechanism of the plaque inhibition by chlorhexidine" Journal of Dental Research 1975, 54, 57-62).

However, chlorhexidine and chlorhexidine salts can suffer from hydrolytic degradation. It is known that in acidic conditions chlorhexidine degrades by two major pathways: one that leads to direct formation of its primary degradation product *p*-chloroaniline (PCA) and the other that leads to indirect formation of *p*-chloroaniline via the formation of the intermediate (*p*-chlorophenyl)biguanide- N-amidino-N'-(*p*-chlorophenyl)urea (PBG-PAU) with loss of ammonia. In alkaline conditions, chlorhexidine degrades by a different single pathway leading indirectly to *p*-chloroaniline. Postulated chlorhexidine degradation mechanisms are set out in (Zong "Studies on the Instability of Chlorhexidine, Part I: Kinetics

and Mechanisms" Journal of Pharmaceutical Sciences 2012, 101, 2417-2427).

The applicant has discovered that this decomposition pathway can occur in mouthwash compositions.

Arginine

The liquid oral care composition that is a mouthwash, mouthrinse or a spray contains arginine or a salt thereof. Arginine is an $\alpha$-amino acid including a guanidine group with the chemical name 2-amino-5-guanidinopentanoic acid:

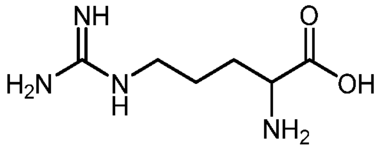

In one embodiment of the invention, the composition includes free arginine.

In one embodiment of the invention, the composition includes a salt of arginine, for example arginine bicarbonate, arginine hydroxide, arginine carbonate or arginine phosphate, or mixtures thereof.

In one embodiment, the liquid oral care composition that is a mouthwash, mouthrinse or a spray comprises 0.1 to 1% w/w, 0.2 to 0.8% w/w or 0.3 to 0.6% w/w arginine or a salt thereof. In particular, the composition comprises 0.4 to 0.5% w/w e.g. 0.5% w/w arginine or a salt thereof.

Surprisingly, the inventors discovered that in an oral care composition that is a mouthwash, mouthrinse or a spray, chlorhexidine decomposition can be reduced by arginine or a salt thereof. In other words, the amount of chlorhexidine that chemically decomposes is less in the presence of arginine or a salt thereof.

By reducing the rate of degradation of chlorhexidine the antibacterial effect of the active ingredient is prolonged and the shelf life of the liquid oral care composition extended (see Example 3).

Zinc salt

The liquid oral care composition that is a mouthwash, mouthrinse or a spray contains a zinc salt.

In one embodiment, the zinc salt is zinc oxide, zinc lactate, zinc chloride, zinc citrate, zinc acetate, zinc borate, zinc butyrate, zinc carbonate, zinc formate, zinc gluconate, zinc glycerate, zinc glycolate, zinc phosphate, zinc picolinate, zinc proprionate, zinc salicylate, zinc silicate, zinc stearate, zinc tartrate, zinc undecylenate, zinc phosphate, zinc ricinoleate, zinc nitrate or zinc sulfate or mixtures thereof.

In one embodiment, the zinc salt is zinc lactate, zinc chloride or zinc citrate, or mixtures thereof.

In one embodiment, the zinc salt is zinc lactate. In one embodiment, the zinc salt is zinc chloride. In one embodiment, the zinc salt is zinc citrate.

In one embodiment, the zinc salt is zinc lactate.

In particular, the zinc salt is zinc acetate. In one embodiment, the zinc acetate is zinc acetate dihydrate.

In one embodiment, the oral care composition that is a mouthwash, mouthrinse or a spray comprises 0.1 to 1% w/w zinc salt. Alternatively, the composition contains 0.2 to 0.9% w/w, 0.3 to 0.8% w/w or 0.4 to 0.7% w/w zinc salt. In a further embodiment, the composition contains 0.2 to 0.5% w/w zinc salt. In a preferred embodiment, the composition contains 0.3% w/w zinc salt.

In one embodiment, the oral care composition that is a mouthwash, mouthrinse or a spray comprises 0.1% to 1% w/w zinc acetate. Alternatively, the composition contains 0.2 to 0.9% w/w, 0.3 to 0.8% w/w or 0.4 to 0.7% w/w zinc acetate. In a further embodiment, the composition contains 0.2 to 0.5% w/w zinc acetate. In a preferred embodiment, the composition contains 0.3% w/w zinc acetate.

Zinc ions also reduce the production of volatile sulfur compounds in the oral cavity. It is thought that a reaction between zinc and sulfur produces a non-volatile sulfide product, thereby preventing the transformation of sulfur containing substrates to volatile sulfur compounds. In addition, zinc ions possess certain antibacterial activity known to inhibit plaque formation and reduce acid formation in dental plaque (see "Oral malodour and active ingredients for treatment", International Journal of Cosmetic Science, 2010, 32, 321-329).

As explained below, the combination of zinc acetate and chlorhexidine provides a synergistic effect against the production of volatile sulfur compounds.

The compositions

The liquid oral care composition is a mouthwash, mouthrinse or a spray. In particular, the liquid oral care composition is a mouthwash, for example an aqueous mouthwash. Accordingly, the composition has a form and consistency that allows the composition to be washed around the mouth and to be sprayed into the mouth,

Other components

The oral care composition that is a mouthwash, mouthrinse or a spray generally includes water. In addition, the oral care composition includes other additives and adjuvants commonly found in oral care compositions, for example an antiplaque agent, a masking agent, a flavoring agent, and a buffering agent.

*Water*

Typically, water (i.e. demineralised water) is present as the balance of the composition. In other words, the composition is an aqueous oral care composition.

*Fluoride*

In one embodiment, the oral care composition that is a mouthwash, mouthrinse or a spray includes one or more fluoride salts.

In one embodiment, the fluoride salt is selected from stannous fluoride, sodium fluoride, potassium fluoride, potassium monofluorophosphate, sodium monoflourophosphate, ammonium monoflourophosphate, sodium flourosilicate, ammonium flourosilicate, amine fluorides such ammonium fluoride, and combinations thereof.

In one embodiment, the oral care composition that is a mouthwash, mouthrinse or a spray includes an amount of a fluoride salt such that the composition includes from 50 to 5000 ppm, 100 to 1000 ppm fluoride ions, 200 to 500 ppm fluoride ions e.g. about 250 ppm fluoride ions.

In one embodiment, the oral care composition that is a mouthwash, mouthrinse or a spray comprises 0.01 to 1% w/w, 0.01 to 0.1% w/w, or 0.02 to 0.07% w/w e.g. 0.01% w/w fluoride salt.

*Masking agent*

In one embodiment, the oral care composition that is a mouthwash, mouthrinse or a spray includes a masking agent, such as tannic acid, glycyrrhizin derivatives, or acesulfame e.g. acesulfame potassium. The masking agent is present in an amount sufficient to reduce the aftertaste of any minerals present in the oral care preparation that is a mouthwash, mouthrinse or a spray. In particular, when present, the composition includes 0.01 to 0.1% w/w masking agent, for example 0.01 to 0.05% w/w masking agent.

*Flavouring agent*

In one embodiment, the oral care composition that is a mouthwash, mouthrinse or a spray includes one or more flavouring agents, such as peppermint, spearmint, cinnamon, wintergreen oils, menthol, or methyl salicylate derivatives. Other flavouring agents may be used individually or in combination. In one embodiment, the composition includes 0.01 to 1% w/w e.g. 0.2 to 0.5% w/w of a flavouring agent.

*Emulsifying agent*

In one embodiment, the oral care composition that is a mouthwash, mouthrinse or a spray includes one or more emulsifying agents, such as PEG-40 hydrogenated castor oil, or polysorbate-20. In one embodiment, the composition includes 0.1 to 10% w/w e.g. 1% w/w of a flavouring agent.

*Buffering agent*

In another embodiment, the oral care composition that is a mouthwash, mouthrinse or a spray includes a buffering agent, such as citric acid, and benzoic acid. In one embodiment, the oral care composition that is a mouthwash, mouthrinse or a spray includes a buffering agent in an amount such that the pH of the oral care preparation is from 5 to 8, for example from 5 to 7, and in particular from 5 to 6.

*Other components*

A variety of additives and adjuvants can be included to make the oral care composition that is a mouthwash, mouthrinse or a spray more amenable for use in a particular end-use application without negatively affecting its efficacy

or the stability of chlorhexidine in a substantial manner. Examples include, but are not limited to, emollients, fragrances, pigments, dyes, flavors, abrasives, bleaching agents, preservatives, antioxidants, and the like.

**Preferred compositions**

Further aspects of the invention

Particular compositions according to the invention include the following.

A liquid oral care composition that is a mouthwash, mouthrinse or a spray comprising:

- 0.005 to 0.2% w/w chlorhexidine or a salt thereof;
- 0.1 to 1% w/w arginine or a salt thereof; and
- 0.1 to 1% w/w zinc salt.

A liquid oral care composition that is a mouthwash, mouthrinse or a spray comprising:

- 0.005 to 0.2% w/w chlorhexidine or a salt thereof;
- 0.1 to 1% w/w arginine or a salt thereof; and
- 0.1 to 1% w/w zinc salt.

wherein the mouthwash, mouthrinse or spray is an aqueous solution, and the aqueous solution has a pH of from 5 to 6.

A liquid oral care composition that is a mouthwash, mouthrinse or a spray comprising:

- 0.005 to 0.2% w/w chlorhexidine or a salt thereof;
- 0.1 to 1% w/w arginine or a salt thereof; and
- 0.1 to 1% w/w zinc acetate.

A liquid oral care composition that is a mouthwash, mouthrinse or a spray comprising:

- 0.005 to 0.2% w/w chlorhexidine or a salt thereof;
- 0.1 to 1% w/w arginine or a salt thereof; and
- 0.1 to 1% w/w zinc acetate,

wherein the mouthwash, mouthrinse or a spray is an aqueous solution, and the aqueous solution has a pH of from 5 to 6.

Further particular compositions according to the invention include the following.

A liquid oral care composition that is a mouthwash, mouthrinse or a spray comprising:

- 0.02 to 0.04% w/w chlorhexidine or a salt thereof;
- 0.3 to 0.6% w/w arginine or a salt thereof; and
- 0.2 to 0.5% w/w zinc salt.

A liquid oral care composition that is a mouthwash, mouthrinse or a spray comprising:

- 0.02 to 0.04% w/w chlorhexidine or a salt thereof;
- 0.3 to 0.6% w/w arginine or a salt thereof; and
- 0.2 to 0.5% w/w zinc salt.

wherein the mouthwash, mouthrinse or spray is an aqueous solution, and the aqueous solution has a pH of from 5 to 6.

A liquid oral care composition that is a mouthwash, mouthrinse or a spray comprising:

- 0.02 to 0.04% w/w chlorhexidine or a salt thereof;
- 0.3 to 0.6% w/w arginine or a salt thereof; and
- 0.2 to 0.5% w/w zinc acetate.

A liquid oral care composition that is a mouthwash, mouthrinse or a spray comprising:

- 0.02 to 0.04% w/w chlorhexidine or a salt thereof;
- 0.3 to 0.6% w/w arginine or a salt thereof; and

- 0.2 to 0.5% w/w zinc acetate,

wherein the mouthwash, mouthrinse or a spray is an aqueous solution, and the aqueous solution has a pH of from 5 to 6.

**Uses of the invention**

Use of the mouthwash

The compositions of the invention include antibacterial agents i.e. chlorhexidine and a zinc salt.
Accordingly, the invention provides the liquid oral care compositions for use in therapy. In particular, the compositions are administered to a patient orally.
In one embodiment, the invention provides the liquid oral care compositions for use in the treatment or prophylaxis of a bacterial infection.
In one embodiment, the invention provides a method of treating a bacterial infection by administering the liquid oral care composition to a patient in need thereof.
The compositions of the invention include agents which reduce the formation of volatile sulfur compounds. Chlorhexidine has an antibacterial effect against the microorganisms that breakdown sulfur containing amino acids. Zinc compounds are thought to react with sulfur containing compounds to give non-volatile sulfur compounds.
Accordingly, the invention provides the use of the liquid oral care compositions for neutralising bad breath i.e. halitosis.

**EXAMPLES**

The invention will now be illustrated by reference to the following examples.

Example 1: Efficacy of combinations (background example)

In vitro experiments were performed to test efficacy of zinc acetate and chlorhexidine to inhibit formation of volatile sulfur compounds (VSC).
10 microlitres of the following solutions were added to a 1 ml sample of freshly collected human saliva in a test tube:

- 0.3% zinc acetate (1);
- 0.025% chlorhexidine (2); and
- 0.025% chlorhexidine + 0.3% zinc acetate (3).

The test tubes were closed with a stopper and incubated overnight at 37°C. A tube which contained saliva only was used as a control. The gaseous VSCs produced by the samples were measured by gas chromatography, and compared to pure samples of hydrogen sulfide and methyl mercaptan.

| Composition | HS | FIC index | MM | FIC index |
|---|---|---|---|---|
| Control | > 27 000 000 | | > 41 000 000 | |
| 1 | 150 000 | | 4 000 000 | |
| 2 | 443 000 | | 355 000 | |
| 3 | 82 000 | 0.33 | 34 000 | 0.01 |

The composition comprising saliva alone (control) contained very high amounts of both hydrogen sulfide (HS) and methyl mercaptan (MM).
The composition which contained zinc acetate alone (1) had lower amounts of both HS and MM. However, zinc acetate reduced the amount of HS much more than the amount of MM.
The composition comprising chlorhexidine alone (2) had lower amounts of both HS and MM.
However, the combinations of zinc acetate and chlorhexidine provided a clear further reduction in VSCs, both for HS and MM.
An examination was conducted to investigate whether the effect of the combination was synergistic. This was performed according to the method of Behrenbaum (J.Inf. Dis. 137:122-130, 1978). The fractional inhibitory concentrations (the FIC index) was calculated based on the amounts (AUC) of hydrogen sulfide and methyl mercaptan formed

under different conditions. A low AUC thus indicates presence of a strong inhibitor. The FIC index was calculated from the following formula: (A+B)/A + (A+B)/B, where A+B represents the combinations of zinc and antibacterial agent, whereas A and B alone represent the individual agents.

A FIC index below 1 indicates a synergistic effect. Accordingly, it can be seen that the combination had a synergistic effect against both HS and MM.

Example 2: Exemplary formulation

An example of a mouthwash according to the invention is as follows (Formulation 1)

| **Ingredient** | **Function** | **% w/w** |
|---|---|---|
| Purified water | Solvent | Balance |
| Sodium fluoride | Antiplaque agent | 0.01-0.1 |
| Chlorhexidine diacetate | Antimicrobial agent | 0.01-0.1 |
| Zinc acetate | Antimicrobial agent | 0.01-0.1 |
| Acesulfame potassium | Masking agent | 0.01-0.1 |
| Hydrogenated starch hydrolysate | Humectant | 1-10 |
| Aroma | Flavoring agent | 0.01-1 |
| PEG-40 hydrogenated castor oil | Emulsifying agent | 0.1-10 |
| Glycerin | Humectant | 1-20 |
| Citric acid | Buffering agent | 0.1-1 |
| Arginine | Stabilising agent | 0.1-1 |
| pH | - | 4-7 |

It will be appreciated that the above composition is only an example, and the invention is not limited to only such compositions.

Example 3: Stability studies

The following simplified formulations were used for these studies:

| Ingredient | % w/w | | | | | | | | | | |
|---|---|---|---|---|---|---|---|---|---|---|---|
| | 1 | 2 | 3 | 4 | 5 | 6 | 7 | 8 | 9 | 10 | 11 |
| Purified water | 79.57 | 77.38 | 69.57 | 67.39 | 72.67 | 77.55 | 69.83 | 77.41 | 77.72 | 77.56 | 77.57 |
| Chlorhexidine diacetate | 0.03 | 0.03 | 0.03 | 0.03 | 0.03 | 0.03 | 0.03 | 0.03 | 0.03 | 0.03 | 0.03 |
| Arginine | 0.5 | 0.5 | 0.5 | 0.5 | - | - | - | - | - | - | - |
| Polyvinylpyrrolidone (PVP) | - | - | - | - | - | - | - | - | 2.00 | - | 2.00 |
| PEG-40 hydrogenated castor oil | 1.00 | 1.00 | 1.00 | 1.00 | 1.00 | 1.00 | 1.00 | 2.40 | 1.70 | 2.40 | 1.70 |

The stability of the formulations was measured by HPLC-UV after storage at 60°C for 42 days. The formulations were stored in PET bottles with PP caps.

Compositions 1-4 include arginine as a stabilising agent. Compositions 5-11 do not include arginine. In particular, compositions 1-4 and compositions 5-7 differ only in the presence or absence of arginine. The data show that arginine improved the stability of the chlorhexidine.

| | Chlorhexidine (mg/mL) | | | | | Chlorhexidine (% difference) | | | |
|---|---|---|---|---|---|---|---|---|---|
| Formulation | Time (days) | | | | | Time (days) | | | |
| | **0** | **14** | **28** | **42** | | **0** | **14** | **28** | **42** |
| 1 | 0.303 | 0.305 | 0.288 | 0.296 | | 100.0 | 100.7 | 95.0 | 97.7 |
| 2 | 0.303 | 0.299 | 0.294 | 0.286 | | 100.0 | 98.7 | 97.0 | 94.5 |
| 3 | 0.314 | 0.307 | 0.291 | 0.296 | | 100.0 | 97.8 | 92.7 | 94.3 |
| 4 | 0.321 | 0.308 | 0.288 | 0.301 | | 100.0 | 96.0 | 89.7 | 93.8 |
| 5 | 0.310 | 0.266 | 0.233 | 0.250 | | 100.0 | 85.8 | 75.2 | 80.6 |
| 6 | 0.304 | 0.309 | 0.279 | 0.261 | | 100.0 | 101.6 | 91.8 | 85.9 |
| 7 | 0.306 | 0.312 | 0.283 | 0.266 | | 100.0 | 102.0 | 92.5 | 87.0 |
| 8 | 0.302 | 0.274 | 0.247 | 0.222 | | 100.0 | 90.7 | 81.8 | 73.5 |
| 9 | 0.313 | 0.249 | 0.205 | 0.155 | | 100.0 | 79.6 | 65.4 | 49.5 |
| 10 | 0.309 | 0.281 | 0.259 | 0.255 | | 100.0 | 90.9 | 83.8 | 82.5 |
| 11 | 0.328 | 0.243 | 0.208 | 0.169 | | 100.0 | 74.1 | 63.5 | 51.6 |

## Claims

1. A liquid oral care composition that is a mouthwash, mouthrinse or a spray comprising:

   • 0.005 to 1% w/w chlorhexidine or a salt thereof;
   • 0.1 to 1% w/w arginine or a salt thereof; and
   • a zinc salt.

2. The oral care composition according to claim 1, comprising 0.2 to 0.8% w/w arginine or a salt thereof.

3. The oral care composition according to claim 1 or 2, comprising 0.005 to 0.1% w/w chlorhexidine or a salt thereof.

4. The oral care composition according to any one of claims 1 to 3, comprising 0.02 to 0.04% w/w chlorhexidine or a salt thereof.

5. The oral care composition according to any one of claims 1 to 4, comprising chlorhexidine in the form of a salt which is chlorhexidine diacetate, chlorhexidine dihydrochloride or chlorhexidine digluconate, or mixtures thereof, for example comprising chlorhexidine in the form of a salt which is chlorhexidine diacetate.

6. The oral care composition according to any one of claims 1 to 5, comprising 0.1 to 1% w/w zinc salt, for example 0.2 to 0.5% w/w zinc salt.

7. The oral care composition according to any one of claims 1 to 6, comprising a zinc salt which is zinc lactate, zinc chloride, zinc citrate, zinc acetate, zinc borate, zinc butyrate, zinc carbonate, zinc formate, zinc gluconate, zinc glycerate, zinc glycolate, zinc phosphate, zinc picolinate, zinc proprionate, zinc salicylate, zinc silicate, zinc stearate, zinc tartrate, zinc undecylenate, zinc phosphate, zinc ricinoleate, zinc nitrate, or zinc sulfate or mixtures thereof, for example comprising a zinc salt which is zinc acetate.

8. The liquid oral care composition according to any one of claims 1 to 7, wherein the liquid oral care composition is

a mouthwash or a mouthrinse.

9. The liquid oral care composition according to any one of claims 1 to 7, wherein the liquid oral care composition is a spray.

10. The oral care composition according to any one of claims 1 to 9, wherein the oral care composition is an aqueous composition.

11. The oral care composition according to claim 10, wherein the aqueous composition has a pH of from 4 to 7, for example from 5 to 6.

12. The oral care composition according to any one of claims 1 to 11, comprising:

- 0.01-0.1% w/w of an antiplaque agent; and/or
- 0.01-1% w/w of a masking agent; and/or
- 0.1-10% w/w of an emulsifying agent; and/or
- 0.1-1% w/w of a buffering agent.

13. The oral care composition according to any one of claims 1 to 12 for use in therapy.

14. The oral care composition for use according to claim 13, for use in the treatment or prophylaxis of a bacterial infection or halitosis.

## Patentansprüche

1. Eine Mundpflegelösung als Mundwasser, Mundspülung oder Spray, bestehend aus:

- 0,005 bis 1Gew.-% Chlorhexidin oder einem Salz davon;
- 0,1 bis 1Gew.-% Arginin oder einem Salz davon und
- einem Zinksalz.

2. Die Mundpflegelösung gemäß Anspruch 1, bestehend aus 0,2 bis 0,8 Gew.-% Arginin oder einem Salz davon.

3. Die Mundpflegelösung gemäß Anspruch 1 oder 2, bestehend aus 0,005 bis 0,1 Gew.-% Chlorhexidin oder einem Salz davon.

4. Die Mundpflegelösung gemäß einem der Ansprüche 1 bis 3, bestehend aus 0,02 bis 0,04 Gew.-% Chlorhexidin oder einem Salz davon.

5. Die Mundpflegelösung gemäß einem der Ansprüche 1 bis 4, bestehend aus Chlorhexidin in Form eines Salzes, das Chlorhexidindiacetat, Chlorhexidindihydrochlorid oder Chlorhexidindigluconat ist, oder Mischungen davon, beispielsweise bestehend aus Chlorhexidin in Form eines Salzes, das Chlorhexidindiacetat ist.

6. Die Mundpflegelösung gemäß einem der Ansprüche 1 bis 5, bestehend aus 0,1 bis 1 Gew.-% Zinksalz, beispielsweise 0,2 bis 0,5 Gew.-% Zinksalz.

7. Die Mundpflegelösung gemäß einem der Ansprüche 1 bis 6, bestehend aus einem Zinksalz, das Zinklactat, Zinkchlorid, Zinkcitrat, Zinkacetat, Zinkborat, Zinkbutyrat, Zinkcarbonat, Zinkformat, Zinkgluconat, Zinkglycerat, Zinkglykolat, Zinkphosphat, Zinkpicolinat, Zinkpropionat, Zinksalicylat, Zinksilikat, Zinkstearat, Zinktartrat, Zink-Unecylenat, Zinkphosphat, Zinkricinoleat, Zinknitrat oder Zinksulfat oder Mischungen davon ist, beispielsweise bestehend aus einem Zinksalz, das Zinkacetat ist.

8. Die Mundpflegelösung gemäß einem der Ansprüche 1 bis 7, wobei die Mundpflegelösung Mundwasser oder Mundspülung ist.

9. Die Mundpflegelösung gemäß einem der Ansprüche 1 bis 7, wobei die Mundpflegelösung ein Spray ist.

**10.** Die Mundpflegelösung gemäß einem der Ansprüche 1 bis 9, wobei die Mundpflegelösung eine wässrige Zusammensetzung ist.

**11.** Die Mundpflegelösung gemäß Anspruch 10, wobei die wässrige Zusammensetzung einen pH-Wert von 4 bis 7, beispielsweise von 5 bis 6, aufweist.

**12.** Mundpflegelösung gemäß einem der Ansprüche 1 bis 11, bestehend aus:

- 0,01 bis 0,1 Gew.-% eines Antiplaque-Wirkstoffs; und/oder
- 0,01 bis 1 Gew.-% eines Maskierungsmittels; und/oder
- 0,1 bis 10 Gew.-% eines Emulgators; und/oder
- 0,1 bis 1 Gew.-% eines Puffermittels.

**13.** Mundpflegelösung gemäß einem der Ansprüche 1 bis 12 zur Verwendung in der Therapie.

**14.** Mundpflegelösung zur Verwendung gemäß Anspruch 13 zur Verwendung bei der Behandlung oder Prophylaxe einer bakteriellen Infektion oder Halitosis.

## Revendications

**1.** Composition liquide de soins bucco-dentaires qui est un bain de bouche, un rince-bouche ou un spray comprenant :

- 0,005 à 1 % p/p de chlorhexidine ou un de ses sels ;
- 0,1 à 1 % p/p d'arginine ou un de ses sels ; et
- un sel de zinc.

**2.** Composition de soins bucco-dentaires selon la revendication 1, comprenant 0,2 à 0,8 % p/p d'arginine ou un de ses sels.

**3.** Composition de soins bucco-dentaires selon la revendication 1 ou 2, comprenant 0,005 à 0,1 % p/p de chlorhexidine ou un de ses sels.

**4.** Composition de soins bucco-dentaires selon l'une quelconque des revendications 1 à 3, comprenant 0,02 à 0,04 % p/p de chlorhexidine ou un de ses sels.

**5.** Composition de soins bucco-dentaires selon l'une quelconque des revendications 1 à 4, comprenant de la chlorhexidine sous la forme d'un sel qui est du diacétaate de chlorhexidine, du dichlorhydrate de chlorhexidine ou du digluconate de chlorhexidine, ou des mélanges de ceux-ci, par exemple comprenant de la chlorhexidine sous la forme d'un sel qui est du diacétate de chlorhexidine.

**6.** Composition de soins bucco-dentaires selon l'une quelconque des revendications 1 à 5, comprenant 0,1 à 1 % p/p de sel de zinc, par exemple 0,2 à 0,5 % p/p de sel de zinc.

**7.** Composition de soins bucco-dentaires selon l'une quelconque des revendications 1 à 6, comprenant un sel de zinc qui est du lactate de zinc, du chlorure de zinc, du citrate de zinc, de l'acétate de zinc, du borate de zinc, du butyrate de zinc, du carbonate de zinc, du formate de zinc, du gluconate de zinc, du glycérate de zinc, du glycolate de zinc, du phosphate de zinc, du picolinate de zinc, du proprionate de zinc, du salicylate de zinc, du silicate de zinc, du stéarate de zinc, du tartrate de zinc, de de l'undécylénate de zinc, du phosphate de zinc, du ricinoléate de zinc, du nitrate de zinc, ou du sulfate de zinc ou des mélanges de ceux-ci, comprenant par exemple un sel de zinc qui est l'acétate de zinc.

**8.** Composition de soins bucco-dentaires liquides selon l'une quelconque des revendications 1 à 7, dans laquelle la composition de soins bucco-dentaires liquides est un bain de bouche ou un rince-bouche.

**9.** Composition de soins bucco-dentaires liquides selon l'une quelconque des revendications 1 à 7, dans laquelle la composition de soins bucco-dentaires liquides est un spray.

**10.** Composition de soins bucco-dentaires selon l'une quelconque des revendications 1 à 9, dans laquelle la composition de soins bucco-dentaires est une composition aqueuse.

**11.** Composition de soins bucco-dentaires selon la revendication 10, dans laquelle la composition aqueuse a un pH de 4 à 7, par exemple de 5 à 6.

**12.** Composition de soins bucco-dentaires selon l'une quelconque des revendications 1 à 11, comprenant :

- 0,01 à 0,1 % p/p d'un agent antiplaque ; et/ou
- 0,01 à 1 % p/p d'un agent de masquage ; et/ou
- 0,1 à 10 % p/p d'un agent émulsifiant ; et/ou
- 0,1 à 1 % p/p d'un agent tampon.

**13.** Composition de soins bucco-dentaires selon l'une quelconque des revendications 1 à 12 pour une utilisation en thérapie.

**14.** Composition de soins bucco-dentaires pour une utilisation selon la revendication 13, pour une utilisation dans le traitement ou la prophylaxie d'une infection bactérienne ou d'une halitose.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 00051559 A
- EP 0181161 A


### Non-patent literature cited in the description


- **VRANI.** Formulation ingredients for toothpastes and mouthwashes. *Bosnian Journal of Basic Medical Sciences,* 2004, vol. 4, 51-58
- **N. LOURITH.** Oral malodour and active ingredients for treatment. *International Journal of Cosmetic Science,* 2010, vol. 32, 321-329
- **RÖLLA ; MELSEN.** On the mechanism of the plaque inhibition by chlorhexidine. *Journal of Dental Research,* 1975, vol. 54, 57-62
- **ZONG.** Studies on the Instability of Chlorhexidine, Part I: Kinetics and Mechanisms. *Journal of Pharmaceutical Sciences,* 2012, vol. 101, 2417-2427
- Oral malodour and active ingredients for treatment. *International Journal of Cosmetic Science,* 2010, vol. 32, 321-329
- **BEHRENBAUM.** *J.Inf. Dis.,* 1978, vol. 137, 122-130